# NEW EUROPEAN PATENT SPECIFICATION

(11) **EP 1 628 642 B2**
(45) Date of publication and mention of the opposition decision: **23.01.2013**
(45) Mention of the grant of the patent: 13.09.2006
(21) Application number: 04741578.1
(22) Date of filing: 14.05.2004
(51) Int. Cl.: A61K 9/20, A61K 9/14

(54) **CONTROLLED DRUG RELEASE COMPOSITION RESISTANT TO IN VIVO MECHANIC STRESS**
KONTROLLIERTE MEDIKAMENTFREIGABE ZUSAMMENSETZUNG MIT IN VIVO MECHANIKSTRESSWIDERSTAND
COMPOSITION POUR LIBERATION CONTROLEE DE MEDICAMENT RESISTANT AU STRESS MECANIQUE IN VIVO

(30) Priority: 14.05.2003 IE 20030366
(43) Date of publication of application: 01.03.2006
(73) Proprietor: Aptalis Pharma Limited, Bray, County Wicklow (IE)
(72) Inventor: DE LUIGI BRUSCHI, Stefano, I-20146 Milano (IT); PENGO, Sergio, I-20030 Bovisio Masciago (IT)
(74) Representative: Ferrari, Barbara
(86) International application number: PCT/EP2004/050814
(87) International publication number: WO 2004/100932

(56) References cited:
- EP-A- 1 262 198
- WO-A-98/10762
- WO-A1-01/78688
- WO-A1-03/035042
- FR-A- 2 656 525
- FR-A- 2 775 597
- US-A- 4 900 755
- US-A- 4 983 400

## Description

### FIELD OF THE INVENTION

The present invention relates to matrixes for drug controlled release characterised by an *in vivo* high mechanical stress resistance and an *in vivo* broad and regular time absorption profile.

### STATE OF THE ART

Swellable matrices are widely used to get monolithic or multiparticulate dosage forms capable of ensuring drug release profile according to the therapeutic needs. A mixture is made dispersing the drug with soluble or insoluble hydrophilic polymers plus compression adjuvants. The mixture is then granulated or directly tabletted to get the final controlled release dosage form. Drug release occurs thanks to the swelling properties of the polymer constituting the matrix that hydrates in presence of aqueous media thus exerting the drug release control.

According to the drug solubility, release mechanism is based on diffusion through the swollen matrix or by polymer erosion or a combination thereof.

Drug release kinetic is governed by several factors i.e. drug solubility, polymer hydration rate, polymer viscosity and loading, type and amount of fillers, etc.

An exhaustive description of these controlled release systems can be found on the US 4,259,314 patent and US 4,680,323 patent.

The matrix systems described in those patents are specifically designed to ensure an *in vitro* drug dissolution rate to give rise to the expected drug peak plasma levels after the intake.

It is well known for those skilled in the art that the choice of the type and quantity of the dissolution medium as well the stirring conditions adopted will depend upon the drug solubility and the absorption window.

In some cases the dissolution method adopted can be used to assess an *in vivo-in vitro* correlation (IVIVC). As a result, knowing the pharmacokinetic of the drug, peak plasma levels can be predicted by the drug dissolution rate data by means of mathematical convolution methods.

Based on the assumption that drug release from a controlled release dosage form is the rate-limiting step in the absorption process, the absorption time profile resulting from the mathematical convolution may be considered to be indicative of an *in vivo* dissolution (D. Young et al. "In vitro-in vivo correlations" advances in experimental medicine and biology, vol. 423 Plenum Press, © 1997 New York and London).

After the intake, the ability of the dosage form manufactured by hydrophilic matrix system to stand the *in vivo* peristalsis, thus maintaining its controlled release properties along the gastro-intestinal tract, is therefore essential to ensure the peak plasma levels expected.

Unfortunately, the *in vivo* poor mechanical resistance conferred to the swollen state by known hydrophilic matrix systems may be the cause of a pharmacokinetic distribution failure even if the *in vitro* drug dissolution rate complies with the defined specifications.

In fact the methodology based on the determination of the drug dissolution rate as a predictive tool to ascertain the *in vivo* peak plasma levels denotes severe limits since the common dissolution apparatus do not stress mechanically the dosage form that maintains a well defined shape during the dissolution test on the contrary, *in vivo* the absorption profile generally shows a dramatics peak plasma level, due to the mechanic smashing of the dosage form. These high drug concentration can lead to undesirable physiological effects.

To get the expected peak plasma levels it becomes of paramount importance to provide the gellable dosage form with suitable mechanical properties to resist the *in vivo* peristalsis without affecting the dissolution properties leading to the desired *in vivo* absorption rate.

It is known from EP 0441245 to include hardened oils (i.e. hardened beef tallow, hardened rapeseed oil) into dosage form in order to increase its mechanical resistance. However the inclusion of these results in a general delaying of the release rate, thus rendering the drug less bioavailable, especially in the early times after administration

FR-A-2 656 525 discloses a controlled drug release composition comprising hydroxypropylmethylcellulose and at least one glyceryl ester. FR-A-2 775 597 discloses pharmaceutical compositions with improved drug bioavaillability comprising glyceryl esters. EP-A-1 262 198 discloses a sustained release composition comprising levadopa, carbidopa and at least one cellulose ether.

All the known controlled release matrixes of the prior art are not completely satisfactory in achieving an *in vivo* mechanical resistance. Therefore the need is felt for improved controlled release matrixes being mechanically resistant against *in vivo* peristalsis and affording a desired release rate *in vivo;* in particular the need is felt to achieve an improved mechanical resistance *in vivo* without incurring the drawback of very prolonged release times, thus maintaining a prompt onset of action soon after administration, and releasing the entire drug dose within acceptable times.

### SUMMARY

The present application relates to a controlled drug release matrix consisting in glyceryl behenate, a cellulose ether and one or more drugs in specific weight ratios.

The cellulose ether is preferably hydroxypropylmethylcellulose, methylcellulose, hydroxypropylcellulose, hydroxyethylcellulose, cellulose acetate, their derivatives or mixture thereof.

Preferably the cellulose ether is characterised by apparent viscosity varying in the range of 15 cP to 100.000 cP (2% w/v aqueous solution, 20°C).

The present matrix is characterised by a mechanical resistance at the swollen state leading to a better prediction of the *in vivo* drug plasma concentration based on the *in vitro* release kinetic studies.

Orally administrable dosage forms can be obtained by processes known per se: e.g. a mixture of cellulose ether, glyceryl ester and one or more drugs can be directly compressed or granulated, than tabletted, etc.

Finally, the present invention relates to the pharmacological exploitations of the described matrixes.

### DESCRIPTION OF THE FIGURES

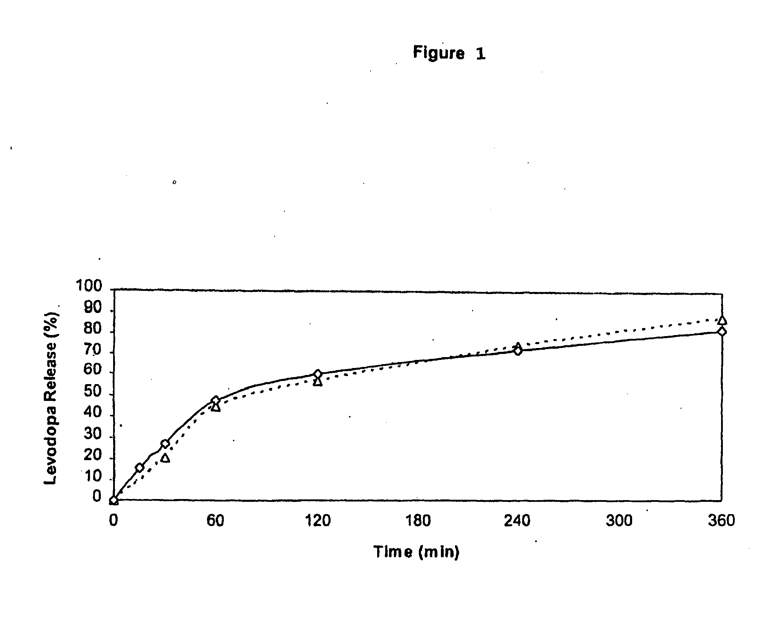

### Figure 1. Dissolution profile of Levodopa from Carbidopa/Levodopa tablets (50/200 mg/tablet)

*In vitro* release amount in percent (%), (vertical axis) during time (hours, horizontal axis) of P003C085 tablets (-◇-) and P003C124 (•••Δ•••) tablets. Tests are carried out using USP XXV Flow through apparatus 4, 16.7 ml/min, gradient, 1 hour HCl 0.1 N, 1 hour HCl 0.01 N, 4 hours BP buffer pH 4, tablets n°=6.

### Figure 2. Levodopa plasma concentrations (fasted, dose: 1 tablet) of Carbidopa/Levodopa 50/200 mg controlled release tablets.

*In vivo* plasma concentration (ng/ml, vertical axis) during time (hours, horizontal axis) of P003C085 controlled release tablet (-o-) and P003C124 controlled release tablet (-□-).

### Figure 3. Mechanical resistance on Levodopa dissolution of Carbidopa/Levodopa 50/200 mg controlled release tablets.

*In vitro* Levodopa release from Carbidopa/Levodopa 50/200 mg controlled release tablets subjected to USP XXV disintegration apparatus, 800 ml BP buffer pH 4.0, tablet n°=3). P003C085 (•••□•••), P003C124 (-◇-).

### DETAILED DESCRIPTION OF THE INVENTION

According to the present invention it has been discovered that, in order to overcome the poor mechanical resistance showed at the swollen state by hydrophilic matrixes, the inclusion in the matrix of glyceryl behenate renders the controlled release system less susceptible to mechanical damages. As a result, during the gastrointestinal transit, the matrix does not lose its mechanical structure thus ensuring an absorption profile governed solely by the dissolution kinetic ensured by the hydro-lipophyilic matrix system.

The mechanical resistance leads to a better overlapping of *in vitro- in vivo* drug release profile, thus enabling a reliable prediction of drug plasma concentration based on *in vitro* dissolution rate.

The matrix composition object of these invention is characterised by a great amount of glyceryl behenate: despite the use of large amount of this lipophilic component, hydro-lipophilic matrixes object of the present invention lead not only to an *in vivo* improvement of mechanical resistance and to a delay of drug absorption profile, but also to a significant release of drug at early stages after administration, thus avoiding a prolonged lag phase before the drug's effects can be perceived by the patient.

Non limiting examples of cellulose ethers are hydroxypropylmethylcellulose, methylcellulose, hydroxypropylcellulose, hydoxyethylcellulose, cellulose acetate, their derivatives or mixture thereof. These cellulose ethers are commercialised in a number of different grades with different apparent viscosity and degree of substitution. Among them, hydroxypropylmethylcellulose (*Methocel* ® *K; Methocel* ® *E*) and methylcellulose *(Methocel* ® *A*) are the polymers preferred. Their apparent viscosity can vary in the range of 15 cP to 100,000 cP (2% w/v aqueous solution, 20°C).

An exhaustive description of the physico-chemical properties of these polymers can be found on the Handbook of Pharmaceutical Excipients, third edition, edited by A.H. Kibbe, © 2000 American Pharmaceutical Association and Pharmaceutical Press.

In particular, it has been surprisingly found that a matrix made of drug, glyceryl behenate and cellulose ether in the weight ratios described below, is capable to associate a very high mechanical resistance to a very quick onset of action immediately after the administration, thereby associating two mutually opposing principles.

In the present matrixes the drug accounts for about 70-95%, the glyceryl ester for about 1-15%, the cellulose ether for about 1-15% by weight of the matrix. Such matrixes are characterised by a sustained drug release profile that leads to a higher plasma concentration during hours far from administration. The matrix is also characterised by releasing significant amounts of drug immediately after administration: therefore the mechanical hardening did not result in delaying the start of the release process; moreover, quite advantageously, this early-stage drug release did not grow into any undesired release peaks.

According to another embodiment, the present invention provides a controlled release matrix containing carbidopa and/or levodopa as a drug, characterised by an ideal drug sustained release and by an exact reproducibility of *in vitro* data when used *in vivo.* In this embodiment the glyceryl behenate and the cellulose ether are mixed with carbidopa and/or levodopa; said carbidopa and/or levodopa represent about 70-95%, preferably 80-95% by weight of the matrix; said glyceryl ester represents about 1-15% by weight of the matrix; said cellulose ether represents about 1 - 15 %of the matrix; the matrix is further characterised by weight ratios of glyceryl behenate to cellulose ether ranging from about 4:1 to 1:1.

Among the method used to estimate the mechanical resistance of controlled release matrices at the swollen state are dynamometric measurements and dissolution kinetic measurement upon stress condition.

Dynamometric tests were made by a texture analyser model TA-XT2 equipped with a 9 mm diameter probe able measure the force that the swollen tablets opposes when pressed.

Dissolution kinetic measurements on stressed conditions were made by USP XXV disintegration apparatus. Tablets were placed in a basket rack immersed in a simulated gastro-intestinal fluid.

Upon the apparatus was operated, liquid aliquots were withdrawn at specific time-points and analysed to assess the drug release.

The matrixes are prepared using pharmaceutical processes namely by direct compression or by granulation processes and final tableting. The process comprises the steps of dispersing one or more drugs with one or more glyceryl esters and one or more cellulose ethers.

The final mixture is than directly compressed or alternatively granulated before being compressed.

In detail, the production stepsare essentially:
- mixing one or more drugs in appropriate amount with glyceryl behenate ester and one or more cellulose ethers;
- direct compression or granulation of the mixture;
- tabletting.

Alternatively drugs can be granulated, then granules are admixed with glyceryl behenate, one or more cellulose ethers: the final mixture is then tabletted.

In another embodiment of the present invention drug/s can be sprayed onto a mixture of the aforementioned components before tabletting, for instance according to the following sequence of steps:
- mixing an appropriate amount of glyceryl behenate with a cellulose ether;
- spraying an appropriate amount of one or more drugs onto the mixture aforementioned;
- compression of the mixture.

The hydro-lipophilic matrix object of the present invention can be monolithic dosage forms such as tablets or multiparticulate dosage forms units such e.g. minitablets, filled into gelatine capsules.

The present invention applies to any acceptable pharmaceutical drug deliverable with controlled release systems.

The invention is further illustrated with the following non limitative examples.

### EXPERIMENTAL SECTION

### Example 1

Two lots of Carbidopa/Levodopa 50/200mg CR tablets were prepared.

Lot P003C085 was made by an hydrophilic matrix, lot P000C124 was made by a hydro-lipophilic matrix, said matrix being object of the present invention.

Their quali-quantitative compositions are illustrated in Table I.

**Table I**

| **Ingredients** | **Lot P003C085 Quantity (mg/tablet)** | **Lot P003C124 Quantity (mg/tablet)** |
|---|---|---|
| 1. Carbidopa monohydrate | 54.0 | 54.0 |
| 2. Levodopa | 200.0 | 200.0 |
| 3. Polyethylenglycol 6000 | 23.0 | 23.0 |
| 4. Carboxyvinylpolymer | 0.6 | --- |
| 5. Polyvinylpyrrolidone K30 | 10.0 | --- |
| 6. Methylcellulose 15 Cp | --- | 14.0 |
| 7. Hydroxypropylmethylcellulose | --- | 1.0 |
| 4000 Cp | | |
| 8. Glyceryl Behenate | --- | 24.0 |
| 9. Silicon dioxide | 2.2 | 3.0 |
| 10.Mg Stearate | 2.2 | 3.0 |

Ingredients 1. and 2. were granulated with 3. Granules were then admixed with the remaining components and the mixtures compressed to get CR tablets having an average hardness of about 100 N. Tablets were subjected to pharmaco-kinetic studies with human volunteers (fasted condition). Because of the pH dependent solubility of the actives, a gradient dissolution analysis was made by the USP XXV flow through apparatus 4. Release mechanism is based on drug diffusion through the swollen polymers and progressive erosion of the matrix.

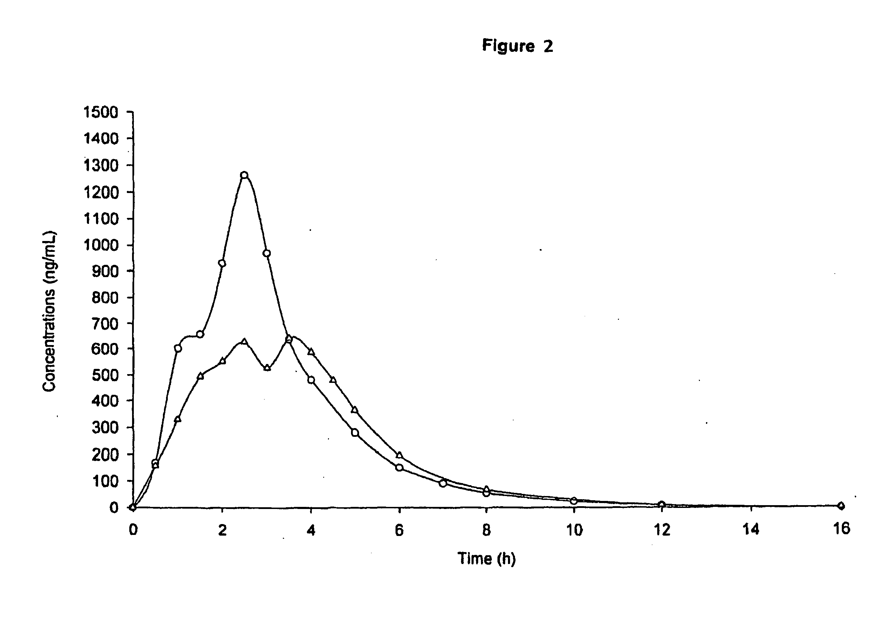

Levodopa *in vitro* release and peak plasma levels are shown in figures 1 and 2. The hydrophilic matrix tablets lot P003C085 and the hydro-lipophilic matrix tablets lot P003C124 showed, at parity of *in vitro* dissolution profile (figure 1), a different *in vivo* time absorption kinetic (figure 2): the prompt and huge peak plasma level evident in hydrophilic tablets lot is absent in hydro-lipophilic tablets lot object of the present invention; on the contrary, in P003C124 tablets the drug plasma concentration after 4 hours is higher than in P003C085 control tablets, thus ensuring a more regular profile, maintaining constant plasma levels for a prolonged time.

To assess mechanical resistance of the two tablets lots, dissolution kinetic measurements on stress conditions were made by the USP XXV disintegration apparatus. A direct comparison was made between the hydrophilic matrix tablets lot P003C085 and the hydro-lipophilic matrix tablets lot P003C124.

Tablets were placed in a basket rack immersed in a simulated gastro-intestinal fluid buffered at pH 4 (BP). Upon the apparatus was operated, liquid aliquots were withdrawn at specific time-points and analysed to assess the drug release.

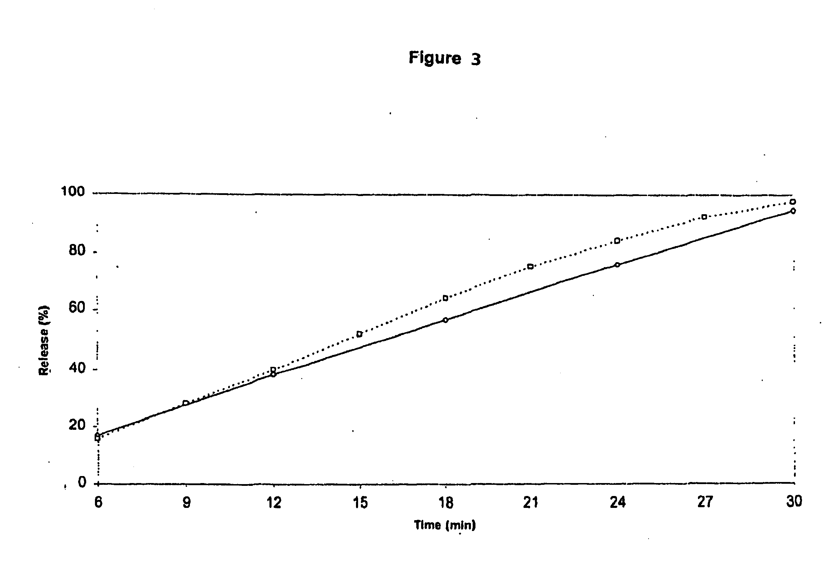

Results are shown in figure 3. The experimental data showed a faster dissolution kinetic for lot P003C085 (•••□•••) due to partial loss of its controlled release properties on stressed conditions. No significant mechanical alterations are observable for lot P003C124 (-◇-).

The higher mechanical resistance ensured by the hydro-lipophilic matrix composition of lot P003C124 did not affected the in vitro dissolution profile (figure 1) thus avoiding significant onset delay on drug absorption (figure 2).

## Claims

1. Controlled drug release matrix consisting in one or more drugs, glyceryl behenate and a cellulose ether, wherein the drug is present in amount of about 70-95% by weight of the matrix, the glyceryl behenate is present in amount of about 1-15% by weight of the matrix, and the cellulose ether is present in amount of about 1-15%.

2. Controlled drug release matrix according to claim 1, wherein the drug is carbidopa and/or levodopa.

3. Controlled drug release matrix according to claim 2, wherein the weight ratio of glycerol behenate to cellulose ether ranges from about 4:1 to 1:1.

4. Controlled drug release matrix according to claims 1-3, wherein the cellulose ether is chosen from hydroxypropylmethylcellulose, methylcellulose, hydroxypropylcellulose, hydroxyethylcellulose, cellulose acetate, their derivatives or mixture thereof.

5. Controlled drug release matrix according to claims 1-4, wherein the cellulose ether is **characterised by** apparent viscosity varying in the range of 15 cP to 100.000 cP (2% w/v aqueous solution, 20°C).

## Patentansprüche

1. Matrix für die kontrollierte Medikamentenfreigabe enthaltend ein Medikament oder mehrere Medikamente, Glycerylbehenat und einen Zelluloseether, wobei das Medikament in einen Mengenmäßigen Anteil vom etwa 70-95% Gew. bezogen auf die Matrix, das Glycerylbehenat in einen Mengenmäßigen Anteil vom etwa 1-15% Gew.- bezogen auf die Matrix, und die Zelluloseether in einen Mengenmäßigen Anteil vom etwa 1-15% vorliegen.

2. Matrix für die kontrollierte Medikamentenfreigabe gamäß Ansprüch 1, wobei es sich bei dem Medikamentenwirkstoff um Carbidopa und/oder Levodopa handelt.

3. Matrix für die kontrollierte Medikamentenfreigabe gamäß Ansprüch 2, wobei das Gewichtsverhältnis von Glycerylbehenat zu Zelluloseether im Bereich von etwa 4:1 bis 1:1 liegt.

4. Matrix für die kontrollierte Medikamentenfreigabe gamäß den Ansprüchen 1-3, wobei der Zelluloseether ausgewählt wirdt unter Hydroxypropylmethylzellulose, Methylzellulose, Hydroxypropylzellulose, Hydroxyethylzellulose, Zelluloseacetat, ihren Derivaten oder Mischungen daraus.

5. Matrix für die kontrollierte Medikamentenfreigabe gamäß den Ansprüchen 1-4, wobei der Zelluloseether durch offensichtliche Viskosität in einem variabel Bereich von 15 cP bis 100.000 cP (2 Gew./Vol.% wässrige Lösung, 20°C) charakterisiert ist.

## Revendications

1. Matrice pour la libération contrôlée de médicaments consistant en un ou plusieurs médicaments, du béhénate de glycéryle et d'un éther cellulosique, dans laquelle le médicament est présent dans une quantité d'environ 70 à 95% en poids par rapport à ladite matrice, le béhénate de glycéryle dans une quantité d'environ 1 à 15 % en poids par rapport à ladite matrice, et l'éther cellulosique dans une quantité d'environ 1 à 15 %.

2. Matrice pour la libération contrôlée de médicaments selon la revendication 1, dans laquelle le médicament est le carbidopa et/ou le lévodopa.

3. Matrice pour la libération contrôlée de médicaments selon la revendication 2, dans laquelle le rapport en poids du béhénate de glycéryle à l'éther cellulosique est dans la gamme d'environ 4:1 à 1:1.

4. Matrice pour la libération contrôlée de médicaments selon les revendications 1-3, dans laquelle l'éther cellulosique est choisi parmi l'hydroxypropylméthylcellulose, la méthylcellulose, l'hydroxypropylcellulose, l'hydroxyéthylcellulose, l'acétate de cellulose, leurs dérivés ou leurs mélanges.

5. Matrice pour la libération contrôlée de médicaments selon les revendications 1-4, dans laquelle l'éther cellulosique est **caractérisé par** une viscosité apparente variable dans la plage allant de 15 cP à 100.000 cP (solution aqueuse à 2% en poids/volume, 20° C).
